# EUROPEAN PATENT APPLICATION

(11) **EP 1 400 238 A1**
(43) Date of publication of application: **24.03.2004**
(21) Application number: 03255971.8
(22) Date of filing: 23.09.2003
(51) Int. Cl.: A61K 7/50

(54) **Compositions for exfoliating skin and treating blackheads**

(30) Priority: 23.09.2002 US 252774
(71) Applicant: Johnson & Johnson Consumer Companies, Inc., Skillman, New Jersey 08858 (US)
(72) Inventor: Walsh, Star Marie, Upper Black Eddy PA 18972 (US)
(74) Representative: Mercer, Christopher Paul

(57) **Abstract**

A skin cleansing composition comprising: from about 1 to about 25 percent by weight of at least one surfactant; from about 0.1 to about 10 percent by weight of particles having a partide size less than 300 microns; from about 0.1 to about 10 percent by weight of particles having a partide size greater than 300 microns; and water is disclosed. The composition cleanses and exfoliates the skin while treating blackheads. A method for cleansing skin and treating blackheads is also disclosed. The method includes applying the compositions of the invention to the skin and rinsing the composition off of the skin.

## Description

### Background of the Invention

### Field of the Invention

The present invention relates to compositions that are useful for exfoliating skin and for treating blackheads. The compositions contain small particles, large particles, and cleansing surfactants.

### Description of the Prior Art

Blackheads can be quite unsightly and frequently causes embarrassment to the sufferer. For this reason, as well as for good hygiene, many people use skin cleansing products on a daily basis. Skin cleansing products include astringents, toners, and the like. Astringent and toner formulations sometimes include anti-acne active ingredients.

Many people also use exfoliating implements or formulations to remove dead skin and make their skin look younger. It would be useful to have a cleansing formulation that treats blackheads and exfoliates the skin simultaneously.

Particles have been incorporated into many commercial cleansers, such as body washes and liquid hand soaps. The particles may be natural, such as pieces of walnut shells. Synthetic particles, such as polyethylene beads have also been incorporated into cleansers.

St. Ives markets several products that contain particles. The products include the Invigorating Apricot Scrub.

For a product to be useful for skin exfoliation and treating blackheads, the particles need to be large enough for exfoliation, but small enough to get into and clean the pores. No such product is known at this time. Therefore, there is a continuing need for a formulation that cleanses and exfoliates the skin while treating blackheads.

### Summary of the Invention

We have discovered that it is possible to provide a skin cleansing product, which exfoliates the skin and treats blackheads simultaneously. The present invention provides a skin cleansing composition including: from about 1 to about 25 percent by weight of at least one surfactant; from about 0.1 to about 10 percent by weight of particles having a particle size less than 300 microns; from about 0.1 to about 10 percent by weight of particles having a particle size greater than 300 microns; and water.

### Detailed Description of Preferred Embodiments

At least one surfactant is included in the compositions of the invention. Anionic, cationic, nonionic, and amphoteric surfactants may be useful.

Types of nonionic surfactants that are suitable for use in this invention include, but are not limited to, the fatty alcohol acid or amide ethoxylates, monoglyceride ethoxylates, sorbitan ester ethoxylates and alkyl polyglycosides. Polysorbate 60 is a preferred nonionic surfactant.

Classes of anionic surfactants useful in this invention include, but are not limited to, the alkyl sulfates, alkyl ether sulfates, sulfosuccinates, isethionates, acyl amides, alkyl ether carboxylates and alkyl phosphates, wherein the alkyl group has from about 6 carbon atoms to about 30 carbon atoms, with about 10 to about 14 carbon atoms being preferred.

Classes of cationic surfactants that are suitable for use in this invention include, but are not limited to, alkyl quatemaries (mono, di, or tri), benzyl quatemaries, ester quatemaries, ethoxylated quaternaries, alkyl amines, and mixtures thereof, wherein the alkyl group has from about 6 carbon atoms to about 30 carbon atoms, with about 8 to about 22 carbon atoms being preferred.

Classes of amphoteric surfactants that are suitable for use in this invention include, but are not limited to, alkylimino-diproprionates, alkylamphoglycinates (mono or di), alkylamphoproprionates (mono or di), alkylamphoacetates (mono or di), N-alkyl β-aminoproprionic acids, alkylpolyamino carboxylates, and phosphorylated imidazolines.

Types of betaines that are suitable for use in this invention include, but are not limited to, alkyl betaines, alkylamido betaines, alkyl sultaines and alkylamido sultaines, wherein the alkyl group has from about 6 carbon atoms to about 30 carbon atoms, with about 10 to about 14 carbon atoms being preferred.

The amount of nonionic surfactant may range from about 0.1 percent by weight to about 10 percent by weight, preferably from about 1 percent by weight to about 6 percent by weight. The amount of anonic surfactant may range from about 0.1 percent by weight to about 5 percent by weight, preferably from about 0.4 percent by weight to about 2 percent by weight. Cationic and amphoteric surfactants may be utilized typically at from about 0.1 percent by weight to about 10 percent by weight.

The compositions of the present invention contain from about 0.1 to about 10 percent by weight of smaller particles having a particle size less than 300 microns. The smaller particles preferably have a particle size less than about 200 microns, more preferably from about 100 microns to about 160 microns. As used herein, particles are solid materials that are insoluble in the compositions of the invention. The particles may be jagged or spherical as is known in the art. The amount of small particles in the formulation preferably ranges from about 1 percent by weight to about 8 percent by weight, preferably from about 2 percent by weight to about 6 percent by weight. Suitable materials for the smaller particles include, but are not limited to, polyethylene, polypropylene, silica, microcrystalline wax, hydrogenated jojoba oil, camuba wax, rye bran wax, and ground shells from nuts, such as ground walnut shells and the like. The smaller particles are preferably jagged.

The compositions of the present invention also contain from about 0.1 to about 10 percent by weight of larger particles having a particle size greater than 300 microns. The larger particles preferably have a particle size from about 350 microns to about 850 microns, more preferably from about 425 microns to about 850 microns. The amount of large particles in the formulation preferably ranges from about 0.5 percent by weight to about 4 percent by weight. Suitable materials for the larger particles include, but are not limited to, polyethylene, polypropylene, silica, microcrystalline wax, hydrogenated jojoba oil, camuba wax, rye bran wax, and ground sheiis from nuts, such as ground walnut shells and the like. The larger particles are preferably spherical.

The skin cleansing compositions of the present invention may include at least one anti-acne active ingredient. Suitable anti-acne active ingredients include. but are not limited to, salicylic acid, sulfur, lactic acid, glycolic add, pyruvic acid, urea, resorcinol, N-acetylcysteine, retinoic acid, benzoyl peroxide, octopirox, triclosan, azelaic acid, phenoxyethanol, phenoxypropanol, flavinoids, derivatives thereof, and combinations thereof. Salicylic acid and benzoyl peroxide are preferred. Salicylic acid is most preferred. The amount of anti-acne active ingredient in the composition of the invention may range from about 0.1 to about 10, preferably from about 1 to about 3 percent by weight, based on the total weight of the composition.

Water is also included in the compositions of the invention. The amount of water utilized will depend on the amounts of the required components and any optional components in the formulation. Generally, the amount of water may range from about 35 to about 99, preferably from about 65 to about 80 percent by weight, based on the total weight of the composition.

The skin cleansing compositions of the invention optionally include humectants, UV absorbers, pH adjusting agents, skin soothers, emollients, preservatives, conditioning agents, thickeners, and fragrances.

Emollients which can be included in the compositions of the invention function by their ability to remain on the skin surface or in the stratum comeum to act as lubricants, to reduce flaking, and to improve the skin appearance. Typical emollients include fatty esters, fatty alcohols, mineral oil, polyether siloxane copolymers and the like. Examples of suitable emollients include, but are not limited to, polypropylene glycol ("PPG")-15 stearyl ether, PPG-10 cetyl ether, steareth-10, oleth-8, PPG-4 lauryl ether, vitamin E acetate, PEG-7 glyceryl cocoate, lanolin, and combinations thereof. PPG-15 stearyl ether is preferred. When utilized, the emollient can be present in an amount from about 1 to about 10, preferably from about 2 to about 8, more preferably from about 4 to about 6 percent by weight, based on the total weight of the composition.

Polyhydric alcohols can be utilized as humectants in the compositions of the invention. The humectants aid in increasing the effectiveness of the emollient, reduce scaling, stimulate removal of built-up scale and improve skin feel. Suitable polyhydric alcohols include, but are not limited to, glycerol (also known as glycerin), polyalkylene glycols, alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-dibutylene glycol, 1,2,6,-hexanetriol, ethoxylated glycerol, propoxylated glycerol and mixtures thereof. Glycerin is preferred. When utilized, the humectant is present in an amount from about 0.1 to about 5, preferably from about 0.1 to about 3 percent by weight, based on the total weight of the composition.

Skin soothers may be added to the compositions of the invention. Suitable skin soothers include, but are not limited to, panthenol, bisabolol, allantoin, and combinations thereof. When utilized, the skin soother is present in an amount from about 0.01 to about 0.75, preferably from about 0.01 to about 0.1 percent by weight, based on the total weight of the composition.

Conditioning agents may also be added to the compositions of the invention. Suitable conditioning agents include, but are not limited to, dimethicone propyl PG-betaine, dimethicone copolyols, polyquatemium-10, and combinations thereof. When utilized, the conditioning agent is present in an amount from about 0.01 to about 1, preferably from about 0.01 to about 0.5 percent by weight, based on the total weight of the composition.

The pH of the compositions of the invention may vary, but typically is from about 3 to about 4, preferably from about 3.25 to about 3.75. The pH may be adjusted through the use of pH adjusters, such as, but not limited to sodium citrate. The amount of pH adjuster utilized will depend on the initial pH of the composition and the volume of composition to be adjusted.

UV absorbers may be added to the compositions of the invention. Suitable UV absorbers include, but are not limited to, benzophenone and its derivatives, cinnamic acid and its derivatives, azoles, imidazoles and the like. When utilized, the amount of UV absorber ranges from about 0.001 to about 0.01 percent by weight, based on the total weight of the composition.

Botanicals, such as aloe barbadensis extract, chamomile extract, thyme extract, rosemary extract, and the like may also be useful in the compositions of the invention. When utilized, botanicals are present at from about 0.01 to about 1, preferably from about 0.01 to about 0.1 percent by weight, based on the total weight of the composition.

Sensates, such as menthyl lactate, menthol, camphor, peppermint, eucalyptus oil, menthoxypropanediol, and the like may also be useful in the compositions of the invention. When utilized, sensates are present at from about 0.01 to about 1, preferably from about 0.05 to about 0.5 percent by weight, based on the total weight of the composition.

Deposition aids may be added to the compositions of the present invention to aid in depositing actives onto the skin. For this purpose, fatty alcohols are useful. Suitable deposition aids include, but are not limited to, stearyl alcohol, cetearyl alcohol, lanolin alcohol, cetyl palmitate, emulsifying wax, blends of cetearyl alcohol & cetearyl sulfate, stearic acid ester of ethoxylated methyl glucoside, glyceryl stearate, and glycol distearate. The amount of deposition aid may range from about 5 percent by weight to about 20 percent by weight, preferably from about 8 percent by weight to about 15 percent by weight.

Preservatives are typically added to compositions to inhibit the growth of microbial organisms. Suitable preservatives to be added to the compositions of the invention include benzoic acid, and Quatemium-15, available commercially as "Dowicil 200" from the Dow Chemical Corporation of Midland, Michigan. Benzoic acid is preferred. When utilized, the preservative is present in an amount from about 0.01 to about 1, preferably from about 0.05 to about 0.5 percent by weight, based on the total weight of the composition.

Any fragrance may be added to the compositions of the invention for aesthetic purposes. Suitable fragrances include, but are not limited to, eucalyptus oil, camphor synthetic, peppermint oil, clove oil, lavendar, chamomile and the like. When utilized, fragrances are present in an amount from about 0.03 to about 3, preferably from about 0.1 to about 0.5 percent by weight, based on the total weight of the composition.

Thickeners may be added to the compositions of the present invention to adjust the viscosity. Suitable thickeners include, but are not limited to, Xanthan gum, Karaya gum, Guar gum, Locust Bean gum, hydroxyethyl cellulose, methyl cellulose, hydroxypropyl cellulose and the like. The amount of thickener may range from about 0.1 percent by weight to about 1 percent by weight, preferably from about 0.25 percent by weight to about 0.75 percent by weight.

Colorants may also be added to the compositions of the invention for aesthetic purposes. Suitable colorants and typical use levels are well known in the art.

The compositions of the invention are useful for cleansing and exfoliating the skin while treating blackheads. The compositions may be supplied as a liquid, which may be applied to the skin utilizing cotton swabs, cloths, and the like. Alternatively, the composition may be absorbed onto wipes, which is known technology, and sold as cleansing wipes. The composition is rinsed off of the skin with water.

An Example is set forth below to further illustrate the nature of the invention and the manner of carrying it out. However, the invention should not be considered as being limited to the details thereof.

### Example 1

A cleansing composition that exfoliates the skin and eliminates blackheads was prepared by admixing the materials in Table 1 in a vessel. The materials were blended until homogenous.

**Table 1**

| Trade Name | INCI Name | Function | Supplier | Amount |
|---|---|---|---|---|
| Water | Purified Water | Vehicle | In House | 69.69 |
| KELTROL 1000 | Xanthan Gum | Viscosifying Agent | Kelco | 0.5 |
| CETAL NF | Cetyl Alcohol | Emulsion Stabilizer | Amerchol | 11.0 |
| BRIJ 721 | Steareth-21 | Surfactant | Uniquema | 2.0 |
| AMPHISOL K | Potassium Cetyl Phosphate | Surfactant | Hoffman La Roche | 0.85 |
| ARLAMOL E | PPG-15 Stearyl Ether | Emollient | Uniquema | 5.0 |
| TWEEN 60 | Polysorbate 60 | Surfactant | Uniquema | 2.0 |
| Salicylic Acid | Salicylic acid | Antiacne Agent | Nipa | 2.0 |
| FRESCOLATE ML | Menthyl Lactate | Cosmetic Sensaid | Haarman and Reimer | 0.15 |
| GREEN CLEAN WW MOD | Fragrance | Fragrance | Givaudan | 0.3 |
| INDUCOS 13/3 | Polyethylene particles 50 - 200 u | Abrasive | Lipo Chemicals | 4.0 |
| FLORABEADS Jojoba 40/60 White | Jojoba Esters | Abrasive | Floratech | 0.01 |
| METABEADS 20/40 Iron Blue | Microcrystalline Wax particles and Ferric ferrocyanide 425 - 850 u | Abrasive | Floratech | 2.5 |

A group of panelists were given samples of the above composition for use at home. After two weeks of use (facial cleansing once a day), the panelists were given a questionairre to determine how they liked the cleanser. The results are shown below in Table 2.

**Table 2**

| Benefit Questioned | Percent Of Panelists That Agreed |
|---|---|
| Helps Unclog Pores | 89 |
| Cleans Deep Down | 96 |
| Helps Get Rid Of Blackheads | 79 |
| Helps Remove Dead Skin Cells | 93 |
| Prevents Blackheads From Forming | 79 |
| Leaves Your Skin Feeling Clean | 96 |
| Removes Tough Blackheads | 70 |
| Removes Trapped Oil And Dirt | 90 |
| Is Effective At Getting Rid Of Blackheads | 73 |

The data above shows that the compositions of the present invention were effective at cleansing and exfoliating the skin while treating blackheads.

## Claims

1. A skin cleansing composition including:
from about 1 to about 25 percent by weight of at least one surfactant;
from about 0.1 to about 10 percent by weight of particles having a particle size less than 300 microns;
from about 0.1 to about 10 percent by weight of particles having a particle size greater than 300 microns; and water.

2. The skin cleansing composition according to claim 1 further comprising from about 0.1 to about 10 percent by weight of an anti-acne active ingredient.

3. The skin cleansing composition according to claim 1 wherein the particle size of the smaller particles is less than about 200 microns and the particle size of the larger particles is from about 350 microns to about 850 microns.

4. The skin cleansing composition according to claim 3 wherein the particle size of the smaller particles is from about 100 microns to about 160 microns and the particle size of the larger particles is from about 425 microns to about 850 microns.

5. The skin cleansing composition according to claim 1 wherein the small particles and
the large particles are made from a material selected from the group consisting of polyethylene, polypropylene, silica, microcrystalline wax, hydrogenated jojoba oil, camuba wax, rye bran wax, and ground shells from nuts.

6. The skin cleansing composition according to claim 5 wherein the small particles are made from polyethylene and the large particles are made from microcrystalline wax.

7. The skin cleansing composition according to claim 6 wherein the amount of small particles ranges from about 2 percent by weight to about 6 percent by weight and the amount of large particles ranges from about 0.5 percent by weight to about 4 percent by weight.

8. The skin cleansing composition according to claim 2 further comprising from about 5 percent by weight to about 20 percent by weight of a deposition aid.

9. The skin cleansing composition according to claim 8 wherein the deposition aid is selected from the group consisting of stearyl alcohol, cetearyl alcohol, lanolin alcohol, cetyl palmitate, emulsifying wax, blends of cetearyl alcohol and cetearyl sulfate, stearic acid ester of ethoxylated methyl glucoside, glyceryl stearate, and glycol distearate.

10. A method of cleansing the skin and treating blackheads comprising:
applying a composition according to claim 1 to the skin, and
rinsing the composition off of the skin.
